# EUROPÄISCHE PATENTSCHRIFT

(11) **EP 1 761 245 B1**
(45) Veröffentlichungstag und Bekanntmachung des Hinweises auf die Patenterteilung: **30.05.2012**
(21) Anmeldenummer: 05819886.2
(22) Anmeldetag: 22.12.2005
(51) Int. Cl.: A61K 9/08, C12C 5/02, C12C 12/00, A23C 9/13, A23F 5/14

(54) **NATÜRLICHER XANTHOHUMOL-HALTIGER EXTRAKT, SOWIE DAS VERFAHREN ZU DESSEN HERSTELLUNG UND DARAUS HERGESTELLTE PRODUKTE**
NATURAL EXTRACT CONTAINING XANTHOHUMOL, AND METHOD FOR THE PRODUCTION THEREOF AND PRODUCTS PRODUCED THEREFROM
EXTRAIT NATUREL CONTENANT DU XANTHOHUMOL, SON PROCEDE DE PRODUCTION ET PRODUITS OBTENUS A PARTIR DUDIT EXTRAIT

(30) Priorität: 22.12.2004 DE 102004063125
(43) Veröffentlichungstag der Anmeldung: 14.03.2007
(73) Patentinhaber: Ta-Xan AG, 65193 Wiesbaden (DE)
(72) Erfinder: BACK, Werner, 85354 Freising (DE); ZÜRCHER, Achim, 4310 Rheinfelden (CH); WUNDERLICH, Sascha, 85354 Freising (DE)
(74) Vertreter: Bobbert, Cornelius
(86) Internationale Anmeldenummer: PCT/EP2005/013916
(87) Internationale Veröffentlichungsnummer: WO 2006/066941

(56) Entgegenhaltungen:
- EP-A- 0 679 393
- WO-A-03/090555
- DE-A1- 10 256 166
- DE-A1- 10 320 250
- US-A1- 2005 019 438

## Beschreibung

### Technisches Gebiet

Die vorliegende Erfindung betrifft ein Verfahren zur Herstellung eines Xanthohumol (XN)-haltigen Röstextrakts aus Röstprodukten von Cerealien, Cerealienmalz oder Kaffee und einem XN-haltigen Hopfenextrakt, sowie dessen Einsatz bei der Getränke- und/oder Lebensmittelherstellung.

### Stand der Technik

Hopfen dient als Rohstoff für die Bierherstellung. Der Hopfen sorgt für die bittere Note des Bieres, aber auch für längere Haltbarkeit aufgrund seiner antibakteriellen Wirkung.

Die wichtigsten Inhaltsstoffe des Hopfens sind, neben den Bittersäuren (z. B. α-Säuren), ätherische Öle, Gerbstoffe und Polyphenole.

Xanthohumol, ein Prenylflavonoid (Polyphenol) des Hopfens, das in den Lupulindrüsen der Hopfendolde vorkommt, hat eine Vielzahl an positiven Wirkungen. Es wird neben der anti-viralen, anti-östrogenen und anti-inflammatorischen Wirkung, auch eine Krebs-chemopräventive Aktivität von XN diskutiert, die derzeit im Tierversuch untersucht wird. Der Xanthohumolgehalt des Hopfens schwankt, je nach Hopfensorte, zwischen 0,1 und 1%. Die Hopfensorten mit einem hohen Xanthohumolgehalt enthalten meist auch einen hohen Anteil an Bitterstoffen.

Hopfenprodukte lassen sich unterteilen in Rohhopfen, Hopfenpellets und Hopfenextrakte. Da Xanthohumol ausschließlich in den Lupulindrüsen der Hopfendolde vorkommt, weisen Hopfenpellets, einen der Anreicherung der α-Säuren entsprechenden, hohen Gehalt an XN auf. Hopfenextrakte können durch Extraktion mit CO₂ und/oder Ethanol gewonnen werden. Während CO₂ Extrakt nahezu kein Xanthohumol enthält, wird bei der Ethanolextraktion von Hopfen XN fast vollständig extrahiert. Neuerdings werden XN-angereicherte Hopfenprodukte durch eine Kombination der beiden Extraktionsverfahren hergestellt. Je nach Herstellungsverfahren werden XN Gehalte zwischen 8 und 99% erreicht. Die Herstellung XN-angereicherter Hopfenextrakte und XN-haltiger Getränke ist, zum Beispiel, in den Patentschriften DE 19939350, DE 10256031, DE 10240065 und EP 1431385 beschrieben.

Im Brauprozess ist Xanthohumol relativ instabil und wird aufgrund seiner begrenzten Löslichkeit weitgehend über den Trub, die Hefe, durch Filtration und Stabilisierung ausgeschieden. Daneben wird Xanthohumol zu Iso-Xanthohumol isomerisiert, welches ebenfalls positive Wirkung zeigt, im Vergleich zu Xanthohumol jedoch in deutlich geringerem Ausmaß. Bei konventioneller Verfahrensweise werden im fertigen Bier meist weniger als 0,2 mg/l XN erreicht. In einzelnen dunklen Bieren vom Typ Stout oder Porter wurden XN-Gehalte von bis zu 1,2 mg/l XN gefunden (Walker et al., Brauwelt 2003). Mit einem speziellen Brauverfahren, das mit späten Hopfengaben und einem raschen Abkühlen der Bierwürze arbeitet, gelingt es, den XN-Gehalt in unfiltrierten hellen Bieren.auf 1-10 mg/l zu steigern (DE 102 56 166).

Ein Vorteil der vorliegenden Erfindung ist, dass durch weitere Verbesserung des Brauverfahrens die Isomerisierung von Xanthohumol zu Iso-Xanthohumol weitgehend unterdrückt wird. Daher ist es möglich höhere Mengen an XN zu dosieren, ohne beispielsweise die Biere unangenehm bitter werden zu lassen. Weiterhin kann die Zugabe von Stabilisierungsmitteln erfolgen, was normalerweise zu einer erheblichen Abnahme des XN-Gehalts führen würde.

Gegenstand der vorliegenden Erfindung ist ein Verfahren zur Herstellung eines XN-haltigen Röstextrakts aus Röstprodukten von Cerealien, Cerealienmalz, Kaffee oder Kakao und einem XN-haltigen Hopfenextrakt. Der XN-haltige Hopfenextrakt zeichnet sich durch einen besonders hohen Gehalt an Xanthohumol aus. Dieser liegt in einem Bereich von 10 mg bis 2 g/kg, bevorzugt in einem Bereich von 200 mg bis 850 mg/kg. Durch den Einsatz dieses Röstextrakts können beispielsweise Biere mit einem, gegenüber dem Stand der Technik, deutlich erhöhtem XN-Gehalt, reinheitsgebotkonform, erhalten werden.

Dabei bezeichnet *XN-haltiger Röstextrakt* einen Extrakt, der aus einem Röstextrakt aus Röstprodukten von Cerealien, Cerealienmalz, Kaffee oder Kakao und aus dem XN-haltigen Hopfenextrakt erhältlich ist. *Röstextrakt* bezeichnet einen kalten oder heißen Anszug aus geschrotetem oder ungeschotetem, geröstetem Malz oder Cerealien und schließt einen heißen oder kalten Auszug von Kaffee oder Kakao ein*; Cerealienmalz* bezeichnet das künstlich, bzw. gesteuerte, zum Keimen gebrachte Getreide, und *XN-haltiger Hopfenextrakt* bezeichnet einen Auszug aus Hopfen, der mittels Lösungsmittel gewonnen wurde und einen erhöhten Gehalt an XN aufweist. *Grundstoffe* ist eine Sammelbezeichnung für einfache oder zusammengesetzte Stoffe, die natürlicher oder synhetischer Herkunft sein können. Sie dienen als Ausgangsmaterial für die weitere Herstellung von Lebensmitteln. In der vorliegenden Erfindung bezeichnen Grundstoffe z. B. Essenzen, die mit XN angereichert werden können, ebenso wie z.B. einen Cola-Grundstoff, gemäß einem Ausführungsbeispiel der vorliegenden Erfindung.

Zur Herstellung von XN-haltigem Röstextrakt wird ein heißer wäßriger Auszug aus den vermahlenen Röstprodukten von, zum Beispiel, Gerste, Weizen, Roggen, sowie den entsprechenden Malzen, Kaffeebohnen oder Kakaobohnen gewonnen. Diesem wird zu Beginn einer Heißhaltephase ein XN-haltiger Hopfenextrakt zugegeben. Die Extraktion von Xanthohumol in den Auszügen der Röstprodukte führt zu einem XN-Gehalt, der weit über der Löslichkeitsgrenze von XN in typischen wässrigen Lösungen liegt, Diese liegt bei einer Wassertemperatur von 23°C bei 1,3 mg/l XN, bei einer Wassertemperatur von 8°C bei 1,1 mg/l XN.

Verantwortlich für die hohen Ausbeuten an Xanthohumol in den Extrakten sind vermutlich lösliche Röstsubstanzen, die XN adsorbieren oder binden und damit scheinbar in Lösung halten. Die Extrakte können ohne nennenswerte XN-Verluste vergoren, eingeengt oder geklärt werden. So wurde einem Ausführungsbeispiel der vorliegenden Erfindung, nach Einengung der XN-haltigen Röstmalzwürze im Extrakt ein Xanthohumolgehalt von etwa 1320 mg/kg erhalten.

Der XN-haltige Röstextrakt kann in der Lebensmittelindustrie eingesetzt werden, so zum Beispiel in der Brau- und Getränkeindustrie, ebenso wie in der Nahrungsmittelindustrie. So könnte der Einsatz des XN-haltigen Röstextrakts die Anreicherung mit Xanthohumol von Spirituosen, Kräutergeist, Kräuterlikör, Wein, Bier, Biermischgetränken oder Magenbitter, aber auch von alkoholfreien, oder alkoholreduzierten Getränken wie Malzgetränken, Kaffee, Kaffee-Ersatz, Kakao, Eistee oder Softdrinks, ebenso wie von Grundstoffen zur Herstellung derartiger Produkte einfach, sicher und kostengünstig gestalten. Als besonderer Vorteil ist zu sehen, dass ein nach diesem Verfahren hergestelltes, XN-angereichertes Röstmalzbier einem konventionell hergestellten Bier zu jedem Zeitpunkt der Bierbereitung, reinheitsgebotkonform, d.h. ohne Verwendung von Emulgatoren oder sonstigen chemischen Zusätzen, zugegeben werden kann.

Eine Anreicherung von Nahrungsmitteln mit Xanthohumol wie, zum Beispiel, Backwaren, Fertigbackmischungen, Brotaufstrichen, Milchprodukten (z.B. Yoghurt, Quark und Milchmischgetränke), Desserts, Eis, Fleischwaren, Saucen oder Kräuterbonbons, sowie die Anreicherung von Xanthohumol in pharmazeutischen Produkten, wie zum Beispiel Hustensaft, ist ebenfalls möglich.

Das Verfahren zur Herstellung eines wässrigen XN-haltigen Extrakts aus Röstprodukten von Cerealien, Cerealienmalz, Kaffee oder Kakao umfasst die folgenden Verfahrensschritte:
a) Zugabe eines XN-haltigen Hopfenproduktes zu einem Auszug, der überwiegend oder vollständig aus Röst- bzw. Farbmalzen oder gerösteter Gerste hergestellt wird; wobei das zugegebene Hopfenprodukt einen XN-Gehalt besitzt, der bezogen auf seine aus dem zu seiner Herstellung eingesetzten Masse mindestens das Fünffache des zu seiner Herstellung eingesetzten Hopfens beträgt; und wobei nach der Zugabe des XN-haltigen Hopfenprodukts zum wässrigen Röstextrakt trotz ursächlicher schlechter Lösbarkeit in wässrigen Lösungen ein Lösen oder sogar ein intensives Lösen von XN erreicht wird;
b) In-Lösung bringen von XN durch ein Vorlösen des XN-haltigen Hopfenproduktes in Ethanol;
c) In-Lösung bringen von XN durch Einrühren, Mischen, Schütteln, Ultraschallbehandlung, Ultraturax-Behandlung oder andere mechanische Kräfte;
d) In-Lösung bringen von XN durch Erhitzen des Röstextraktes;
e) In-Lösung bringen von XN durch Anlegen einer Wechelstromspannung;
f) die Höhe der Zugabe des XN-haltigen Hopfenproduktes beträgt mindestens 50 mg XN pro Liter.

Wie aus den nachfolgenden Beispielen ersichtlich, ist es nicht erforderlich, dass sämtliche der vorstehend angegebenen Verfahrensschritte bei der Umsetzung realisiert werden. Die Verfahrensschritte b) bis h) sind optional.

Im Folgenden werden Beispiele erfindungsgemäßer Verfahren und dadurch hergestellte Getränke gegeben.

### Beispiel 1

Herstellung von Bierwürze aus einer Spezialmalzmischung, bestehend aus 1,9 kg geröstetem Gerstenmalz (Carafa™, Typ 2, Fa. Weyermann, Bamberg) und 0,1 kg Pilsener Malz. Nach einem Kongressmaischverfahren und dem Läutern wurde während des Kochens der Röstmalzwürze mit einer Pfannevollwürzekonzentration von 11%-GG (Gewichts-/Gewichtsprozent) und einer Würzfarbe von 1650 EBC (European Brewing Convention) wurden 25 g/l eines XN-angereicherten Hopfenextrakts zugegeben (XanthoExtrakt, Simon H. Steiner Hopfen GmbH, XN 2,0%). Nach Heißtrub- und Kieselgurabtrennung wurde die XN-haltige Röstmalzwürze im Vakuum (200 mbar, ca. 55°C) auf einen Extraktgehalt von etwa 50% GG eingedampft. Der sirupartige Extrakt ("Dichte 1,25" kg/l) wies eine Farbe von etwa 7500 EBC und einen XN-Gehalt von etwa 1320 mg/kg auf.

### Beispiel 2

Herstellung von Bierwürze aus einer Spezialmischung, bestehend aus 0,05 kg Pilsener Malz und 0,95 kg geröstetem Gerstenmalz (Carafa™, Weyermann). Nach einem Kongressmaischverfahren und dem Läutern wurde die Würze 2 Stunden gekocht. Zu Kochbeginn wurden 5000 mg/l XN zur Würze dosiert. Dabei wurde XN-haltiger Hopfenextrakt aus 80% Kieselgur und 20% Hopfenextrakt (XanthoExtrakt, Hopsteiner, XN 1,7%).verwendet. Neben XN enthält dieser Extrakt auch α-Säuren und Hartharze, die den Geschmack beeinflussen. Die Ausschlagwürze in einer Konzentration von 11,7% GG wurde in 2 Tagen mit untergäriger Bierhefe vergoren und nach 5 Tagen Lagerung zentrifugiert. Das resultierende Röstmalzbier (RMB) wies eine Farbe von 1850 EBC und einen XN-Gehalt von 390 mg/l auf.

### Beispiel 3

Verfahren zur Herstellung eines XN-haltigen Röstmalzextrakts gemäß Beispiel 1, jedoch wurde die Würze vor dem Eindampfen mit Bierhefe vergoren, zentrifugiert und mit Kieselgur filtriert. Das so hergestellte Röstmalzbier, mit einer Stammwürze von 12% und einer Farbe von etwa 1700 EBC wies einen XN-Gehalt von 246 mg/l auf.

### Beispiel 4

Verfahren zur Herstellung eines XN-haltigen Röstmalzbieres gemäß Beispiel 3, jedoch wurde das RMB nach der Filtration im Vakuum (200 mbar, ca. 55°C) auf einen Extraktgehalt von etwa 48% eingedampft. Die Dichte des sirupartigen XN-haltigen Röstmalzbieres lag bei etwa 1,2 kg/l, die Farbe bei 6900 EBC und der XN-Gehalt bei 846 mg/kg.

### Beispiel 5

Verfahren zur Herstellung eines XN-haltigen Röstmalzextrakts gemäß Beispiel 1, jedoch wurde eine Menge von etwa 100 mg XN pro Liter Würze (11% GG Röstmalzextrakt) dosiert. Die weitere Verarbeitung gemäß Beispiel 3 und 4 führte zu einem Röstmalzbier mit einem Extraktgehalt von 47% GG, einer Dichte von 1,2 kg/l, einer Farbe von 7200 EBC und einem XN-Gehalt von 341 mg/l.

### Beispiel 6

Herstellung eines Kaffeeauszugs aus 40 g gemahlenem Röstkaffee und 1 Liter heißem Wasser. Nach Abtrennung des Kaffeesatzes wurden dem heißen Kaffee-Extrakt 100 mg eines XN-haltigen Hopfenextrakts (XN-Gehalt 80%) zugegeben. Es folgte ein intensives Einmischen des Hopfenextrakts bei Temperaturen von über 60°C und ein weiteres Lösen bei 8°C für 24 Stunden. Nach Filtration des XN-haltigen Kaffeegetränks über einen Faltenfilter wies das Kaffeegetränk einen XN-Gehalt von über 12 mg/l auf.

### Beispiel 7

Verfahren zur Herstellung eines XN-haltigen Kaffeegetränks gemäß Beispiel 6, jedoch wurde das gefilterte Kaffeegetränk im Gefriertrockner auf einen Wassergehalt von unter 10% getrocknet. Das lösliche XN-haltige Kaffeegetränks wies einen XN-Gehalt von 1042 mg/kg auf.

### Beispiel 8

Etwa 1,8 g lösliches, XN-angereichertes Kaffeegetränk gemäß Beispiel 7 wurde mit 150 ml heißem Wasser ausgemischt. Der XN-Gehalt des ausgemischten Kaffeegetränks lag bei etwa 10 mg/l.

### Beispiel 9

Einem ungefilterten hellen Lagerbier (XN-Gehalt 0,1 mg/l) wurden 2 g/l des XN-haltigen Röstmalzbieres aus Beispiel 4 zugegeben. Im blanken Bier war gegenüber dem Vergleichsbier eine Farbzunahme von 15 EBC und eine XN-Anreicherung von 1,8 mg/l zu verzeichnen.

### Beispiel 10

Einem unfiltrierten hellen Lagerbier (XN-Gehalt 0,1 mg/l) wurden 20 g/l des XN-haltigen Röstmalzbieres aus Beispiel 4 zugegeben. Das filtrierte Bier wies eine Farbe von 120 EBC und einen XIV-Gehalt von 13 mg/l auf

### Beispiel 11

Einem filtrierten hellen Lagerbier (XN-Gehalt 0,05 mg/l) wurden 20 g/l des XN-haltigen Röstmalzbieres aus Beispiel 4 zugegeben. Das ausgemischte filtrierte Bier wies eine Farbe von 125 EBC und einen XN-Gehalt von 15 mg/l auf.

### Beispiel 12

Einem typischen Cola-Grundstoff, bestehend aus zwei Komponenten, zum einen einer Cola-Essenz, Zuckercouleur und Koffein und zum anderen Phosphorsäure, werden 880 g eines XN-angereicherten Röstmalzbiers aus Beispiel 1 (Konzentration 1320 mg XN/kg) zu 3,05 kg Cola-Grundstoff zugesetzt und homogenisiert. Nach Ausmischung des angereicherten Grundstoffs mit aufbereitetem Wasser (3:1000), Kohlendioxid (6,2 g/l) und Zucker (110 g/l) konnte im trinkfertigen Getränk ein Xanthohumolgehalt von 1,1 mg/l zu verzeichnen.

### Beispiel 13

Ein Kilogramm probiotischer Joghurt wird mit 2,29 g XN-haltigem Röstmalzextrakt aus Beispiel 1 versetzt und homogenisiert. Im pasteurisierten Produkt konnte ein Xanthohumolgehalt von 0,3 mg/100 g festgestellt werden.

### Beispiel 14

Einem pharmazeutischen Produkt, hier 1 Liter Hustensaft, wurde XN-haltiger Röstmalzextrakt (7,6 g) aus Beispiel 1 zugemischt. Das fertige Produkt enthielt etwa 1,0 mg XN/100 ml.

## Patentansprüche

1. Xanthohumol (XN)-haltiger Röstextrakt mit einem XN-Gehalt von wenigstens 10 mg/kg, erhältlich aus einem Röstextrakt, welcher einen kalten oder heißen Auszug aus geschrotetem oder ungeschrotetem, geröstetem Malz, Cerealien, Kaffee oder Kakao umfasst, und einem XN-haltigen Hopfenextrakt.

2. XN-haltiger Röstextrakt nach Anspruch 1, **dadurch gekennzeichnet, dass** der XN-Röstextrakt einen XN-Gehalt in einem Bereich von 50 mg/kg bis 1320 mg/kg, insbesondere in einem Bereich von 200 mg/kg bis 850 mg/kg aufweist.

3. Pharmazeutische Zusammensetzung, **dadurch gekennzeichnet, dass** sie einen XN-haltigen Röstextrakt gemäß einem der Ansprüche 1 oder 2 enthält.

4. Pharmazeutisches Produkt nach Anspruch 3, **dadurch gekennzeichnet, dass** die pharmazeutische Zusammensetzung ein Hustensaft ist.

5. Lebensmittel, **dadurch gekennzeichnet, dass** es einen XN-haltigen Röstextrakt gemäß einem der Ansprüche 1 oder 2 enthält.

6. Lebensmittel nach Anspruch 5, **dadurch gekennzeichnet, dass** das Lebensmittel alkoholische, alkoholreduzierte oder nicht-alkoholische Getränke umfasst.

7. Lebensmittel nach Anspruch 6, **dadurch gekennzeichnet, dass** die alkoholischen oder alkoholreduzierten Getränke, Getränke wie Spirituosen, Kräutergeist, Kräuterliköre, Magenbitter, Wein, Bier oder Biermischgetränke umfassen.

8. Lebensmittel nach Anspruch 6, **dadurch gekennzeichnet, dass** die nichtalkoholischen Getränke, Getränke wie Softdrinks, Kaffeegetränke, Kaffee-Ersatz, Malzgetränke, Eistee oder Grundstoffe für deren Herstellung umfassen.

9. Lebensmittel nach Anspruch 5, wobei das Lebensmittel Backwaren, Brotaufstrich, Milchprodukte, Süßspeisen, Saucen und Fleischwaren umfasst.

10. Lebensmittel nach einem der Ansprüche 5-9, wobei der XN-Gehalt 10 mg/l bis 400 mg/l beträgt, bevorzugt von 12 mg/l bis 390 mg/l, besonders bevorzugt 15 mg/l bis 246 mg/l.

11. Verfahren zur Herstellung eines XN-haltigen Röstextrakts mit einem XN-Gehalt von wenigstens 10 mg/kg, wobei ein XN-haltiger Hopfenextrakt mit einem kalten oder heißen Auszug aus geschrotetem oder ungeschrotetem, geröstetem Malz, Cerealien, Kaffee oder Kakao vermischt und nachfolgend eingeengt wird.

12. Verfahren nach Anspruch 11, wobei der zugegebene XN-haltige Hopfenextrakt einen Gehalt an XN aufweist, der in einem Bereich von 0,5 bis 99 GG-% XN liegt.

13. Verfahren nach Anspruch 11, wobei der XN-haltige Röstextrakt auf eine Trockensubstanz von 40-50 % GG (Gewichts-/Gewichtsprozent), insbesondere 47-48 % GG eingeengt wird.

14. Verfahren nach Anspruch 13, wobei das Einengen durch Eindampfen, Gefriertrocknen oder bei reduziertem Druck erfolgt.

15. Verfahren nach einem der Ansprüche 11 bis 14, wobei der XN-haltige Röstextrakt mit Hefe vergoren und geklärt wird.

16. Verfahren nach Anspruch 15, wobei das Klären durch Zentrifugieren oder Filtrieren erfolgt.

17. Verfahren nach einem der Ansprüche 15 und 16, wobei das XN-angereicherte Röstmalzbier auf einen Extraktgehalt von 40-50 % GG, insbesondere 47-48 % GG eingeengt wird.

18. Verfahren nach Anspruch 11, wobei zur Lösung des XN der Hopfenextrakt in Ethanol vorgelöst wird.

19. Verfahren nach Anspruch 18, wobei das Lösen des XN durch Einrühren, Mischen, Schütteln, Ultraschallbehandlung und/oder Behandlung mit Dispergiergeräten erfolgt.

20. Verfahren nach einem der Ansprüche 18 und 19, **dadurch gekennzeichnet, dass** das Lösen des Xanthohumols ferner Erhitzen und/oder das Anlegen einer Wechselspannung umfasst.

21. Verfahren zur Herstellung eines wässrigen XN-haltigen Extraktes aus Röstprodukten von Cerealien, Cerealienmalz oder Kaffee mit einem XN-Gehalt von über 10 mg/kg, welches die Verfahrensschritte umfasst:
a) Zugeben eines XN-haltigen Hopfenproduktes zu einem Auszug, der überwiegend oder vollständig aus Röst- bzw. Farbmalzen oder gerösteter Gerste hergestellt wird; wobei das zugegebene Hopfenprodukt einen XN-Gehalt besitzt, der bezogen auf seine aus dem zu seiner Herstellung eingesetzten Masse mindestens das Fünffache des zu seiner Herstellung eingesetzten Hopfens beträgt; und wobei nach der Zugabe des XN-haltigen Hopfenproduktes zum wässrigen Röstextrakt trotz ursächlicher schlechter Lösbarkeit in wässrigen Lösungen ein Lösen oder sogar ein intensives Lösen von XN erreicht wird;
wobei das Verfahren ferner **dadurch gekennzeichnet ist, dass** es einen oder mehrere der nachfolgenden Schritte umfasst:
b) In-Lösung bringen von XN durch ein Vorlösen des XN-haltigen Hopfenproduktes in Ethanol;
c) In-Lösung bringen von XN durch Einrühren, Mischen, Schütteln, Ultraschallbehandlung, Ultraturax-Behandlung oder andere mechanische Kräfte;
d) In-Lösung bringen von XN durch Erhitzen des Röstextraktes;
e) In-Lösung bringen von XN durch Anlegen einer Wechselstromspannung ;
f) Zugeben des XN-haltigen Hopfenproduktes in Höhe von mindestens 50 mg XN pro Liter.

22. Verfahren zur Herstellung von XN-haltigem Röstextrakt gemäß Anspruch 21, im Weiteren **dadurch gekennzeichnet, dass** der XN-angereicherte Extrakt auf eine Trockensubstanz von etwa 50 % eingedampft wird.

23. Verfahren zur Herstellung von XN-haltigem Röstextrakt gemäß Anspruch 21, im weiteren **dadurch gekennzeichnet, dass** a) der XN-angereicherte Extrakt mit Hefe vergoren, zentrifugiert oder filtriert wird; b) das XN-angereicherte Röstmalzbier auf einen Extraktgehalt von z.B. 50 % eingedampft wird.

24. Verfahren zur Herstellung von XN-haltigem Extrakt gemäß Anspruch 21 bis 23, im Weiteren **dadurch gekennzeichnet, dass** ein handelsüblicher Röstmalzextrakt bzw. handelsübliches Röstmalzbier zum Lösen des XN-angereicherten Hopfenextraktes verwendet wird.

25. Verfahren zur Herstellung eines XN-haltigen Röstextraktes gemäß Anspruch 21 oder 22, im Weiteren **dadurch gekennzeichnet, dass** ein Auszug aus handelsüblichem Röstkaffee bzw. handelsüblichem löslichen Kaffee zum Lösen des XN-angereicherten Hopfenextraktes verwendet wird.

26. Ein Xanthohumol-haltiger Röstextrakt mit einem XN-Gehalt über 10 mg/kg, dessen Herstellung gemäß den Ansprüchen 21 bis 25 erfolgt. Die Verwendung dieses Extraktes ist für die natürliche Anreicherung von XN in Getränken, wie z.B. Bier, Biermischgetränke, Wein, Kaffeegetränke, Kaffee-Ersatz oder einem anderen alkoholfreien Getränk oder einer Vorstufe dieser Produkte, vorgesehen.

27. Ein Xanthohumol-haltiger Röstextrakt mit einem XN-Gehalt über 10 mg/kg, dessen Herstellung gemäß einem der Ansprüche 21 bis 25 erfolgt. Die Verwendung dieses Extraktes ist für die Herstellung von Xanthohumol-haltigen Backwaren, Brotaufstrich, Desserts, Eis, Fleischwaren, Saucen und ähnlichen Produkten oder einer Vorstufe dieser Produkte vorgesehen.

28. Ein Xanthohumol-haltiger Röstextrakt mit einem XN-Gehalt über 50 mg/kg, dessen Herstellung gemäß einem der Ansprüche 21 bis 25 erfolgt.

29. Ein Xanthohumol-haltiger Röstextrakt mit einem XN-Gehalt über 200 mg/kg, dessen Herstellung gemäß einem der Ansprüche 21 bis 25 erfolgt.

30. Ein Xanthohumol-haltiger Röstextrakt mit einem XN-Gehalt über 200 mg/kg.

## Claims

1. Roasted Extract comprising xanthohumol (XN) with an XN content of at least 10 mg/kg, obtainable from a roasted extract that includes a cold or hot extract of coarsely ground or non-ground roasted malt, cereals, coffee or cacao and a hop extract comprising XN.

2. Roasted extract comprising XN according to claim 1, **characterised in that** said XN content is in a range of 50 mg/kg to 1320 mg/kg, in particular in a range of 200 mg/kg to 850 mg/kg.

3. Pharmaceutical composition, **characterised in that** it comprises a roasted extract comprising XN according to any one of claims 1 or 2.

4. Pharmaceutical product according to claim 3, **characterised in that** the pharmaceutical composition is a cough syrup.

5. Food product, **characterised in that** it comprises the roasted extract comprising XN according to any one of claims 1 or 2.

6. Food product according to claim 5, **characterised in that** the food product is selected from the group consisting of alcoholic, alcohol reduced and non-alcoholic beverages.

7. Food product according to claim 6, **characterised in that** the alcoholic or alcohol reduced beverages are selected from the group consisting of spirits, herbal spirits, herbal liqueurs, bitters, wine, beer, and mixed beverages comprising beer.

8. Food product according to claim 6, **characterised in that** the non-alcoholic beverages are selected from the group consisting of soft drinks, coffee beverages, coffee substitutes, malt beverages, ice tea and basic materials for producing the same.

9. Food product according to claim 5, wherein the food product is selected from the group consisting of bakery products, parfaits, dairy products, sweets, sauces and meat products.

10. Food product according to any one of claims 5 to 9, wherein the XN content is 10 mg/l to 400 mg/l, preferably from 12 mg/l to 390 mg/l, more preferably from 15 mg/l to 246 mg/l.

11. Method for producing a roasted extract comprising XN that has an XN content of at least 10 mg/kg, wherein a hop extract comprising XN is mixed with a cold or hot extract from a coarsely ground or non-ground roasted malt, cereal, coffee or cacao and which is concentrated thereafter.

12. Method according to claim 11, wherein the added hop extract comprising XN has a content of XN which is in a range from 0.5 to 99% w/w (weight/weight percent) of XN.

13. Method according to claim 11, wherein the roasted extract comprising XN is concentrated to dry matter of 40-50% w/w (weight/weight percent), particularly to 47 to 48% w/w (weight/weight percent).

14. Method according to claim 13, wherein the concentrating is performed by evaporating, freeze drying or at reduced pressure.

15. Method according to any one of claims 11 to 14, wherein the roasted extract comprising XN is fermented by yeast and clarified.

16. Method according to claim 15, wherein the clarifying is effected by centrifuging or filtrating.

17. Method according to any one of claims 15 and 16, wherein the method yields a roasted malt beer enriched with XN, which is concentrated to an extract content of 40-50% w/w (weight/weight percent) in particular to 47-48%w/w.

18. Method according to claim 11, wherein the hop extract is pre-dissolved in ethanol for dissolving the XN.

19. Method according to claim 18, wherein the XN is dissolved by at least one of the following: agitating, mixing, shaking, super sonic treatment or treatment with a dispersing means.

20. Method according to any one of claims 18 and 19, **characterised in that** the dissolving of xanthohumol further includes at least one of the following: heating or the application of an alternating current.

21. Method for producing an aqueous extract comprising XN from roasted products of a cereal, cereal malt, or coffee that have an XN content of more than 10 mg/kg, **characterised in that**:
a) the hop product comprising XN is added to an extract which is mainly or completely produced from roasted or colour malt, respectively, or roasted barley; in which
the added hop product has an XN content which, related to its mass used for its production, is at least five times that of the hop used for its production; and in which
dissolving or even an intensive dissolving of XN is achieved after the addition of the hop product comprising XN to the aqueous roasted extract despite the originally low solvability in aqueous solutions;
in which the method is further **characterised by** at least one of the following:
b) dissolving of XN by pre-dissolving of the hop product comprising XN in ethanol;
c) dissolving of XN by agitating, mixing, shaking, supersonic treatment, Ultraturax treatment or any other mechanical forces;
d) dissolving of XN by heating of the roasted extract;
e) dissolving of XN by applying an alternating current;
f) adding of the hop product comprising XN with at least 50 mg of XN per litre.

22. Method For producing the roasted extract comprising XN according to claim 21, further **characterised in that** the extract enriched with XN is concentrated to a dry matter of approximately 50%.

23. Method for producing the roasted extract comprising XN according to claim 23, further **characterised by**:
a) the extract enriched with XN is fermented by yeast, centrifuged or filtered;
b) the method yields a roasted malt beer enriched with XN, which is concentrated to an extract content of e.g. 50%.

24. Method for producing the extract comprising XN according to any one of claims 21 to 23, further **characterised in that** a commercially available roasted malt extract or a commercially available roasted malt beer respectively is used for dissolving the hop extract enriched with XN.

25. Method for producing a roasted extract comprising XN according to any one of claims 21 or 22, further **characterised in that** an extract from a commercially available roasted coffee or from a commercially available instant coffee respectively is used for dissolving the hop extract enriched with XN.

26. Roasted extract comprising xanthohumol with an XN content of more than 10 mg/kg which is produced according to any one of claims 21 to 25, wherein the use of this extract is intended for the natural enrichment ofXN in a beverage selected from the group consisting of beer, a mixed beverage comprising beer, wine, a coffee beverage, a coffee substitute or another non-alcoholic beverage or a precursor of these products.

27. Roasted extract comprising xanthohumol with an XN content ofmore than 10 mg/kg which is produced according to any one of claims 21 to 25, wherein the use of this extract is intended for the production of xanthohumol comprising bakery products, parfaits, desserts, ice cream, meat products, sauces, and similar products or a precursor of these products.

28. Roasted extract comprising Xanthohumol with an XN content of more than 50 mg/kg which is produced according to any one of claims 21 to 25.

29. Roasted extract comprising xanthohumol with an XN content of more than 200 mg/kg which is produced according to any one of claims 21 to 25.

30. Roasted extract comprising xanthohumol with an XN content of more than 200 mg/kg.

## Revendications

1. Extrait torréfié contenant du xanthohumol (XN) ayant une teneur en XN d'au moins 10 mg/kg, que l'on peut obtenir à partir d'un extrait torréfié, qui contient un extrait froid ou chaud de céréales, café, cacao ou malté torréfié concassé ou non concassé, de même qu'un extrait de houblon contenant du XN.

2. Extrait torréfié contenant du XN selon la revendication 1, **caractérisé en ce que** l'extrait torréfié XN présente une teneur en XN dans une fourchette de 50 mg/kg à 1320 mg/kg, en particulier dans une fourchette de 200 mg/kg à 850 mg/kg.

3. Composition, pharmaceutique, **caractérisée en ce qu'**elle comporte un extrait torréfié contenant du XN selon l'une des revendications 1 ou 2.

4. Produit pharmaceutique selon la revendication 3, **caractérisé en ce que** la composition pharmaceutique est un sirop contre la toux.

5. Aliments **caractérisés en ce qu'**ils comportent un extrait torréfié contenant du XN selon l'une des revendications 1 ou 2.

6. Aliments selon la revendication 5, **caractérises en ce que** les aliments comprennent des boissons alcoolisées, faiblement alcoolisées ou sans alcool.

7. Aliments selon la revendication 6, **caractérisés en ce que** les boissons alcoolisées ou faiblement alcoolisées regroupent des boissons comme les spiritueux, l'eau-de-vie herbicinale, les liqueurs herbicinales, les liqueurs digestives, le vin, la bière ou les panachés.

8. Aliments selon la revendication 6, **caractérises en ce que** les boissons sans alcool regroupent des boissons comme les sodas, les boissons à base de café, l'ersatz de café, les boissons à base de malt, le thé glacé ou les ingrédients nécessaires à leur fabrication.

9. Aliment selon la revendication 5, dans lequel les aliments regroupent les pâtisseries, les produits à tartiner, les produits laitiers, les entremets sucrés, les sauces et les viandes.

10. Aliments selon l'une des revendications 5 à 9, où la teneur en XN est de 10 mg/l à 400 mg/l, de préférence de 12 m/l à 390 mg/l, de préférence particulière de 15 mg/l à 246 mg/l.

11. Procédé de fabrication d'un extrait torréfié contenant du XN, ayant une teneur en XN d'au moins 10 mg/kg, dans lequel un extrait de houblon contenant du XN est mélangé à un extrait froid ou chaud de céréales, café, cacao ou malté torréfié concassé ou non concassé, avant concentration.

12. Procédé selon la revendication 11, où l'extrait de houblon contenant du XN ajouté présente une teneur en XN située dans une fourchette de 0,5 à 99 poids/poids en pourcentage de XN.

13. Procédé selon la revendication 11, où l'extrait torréfié contenant du XN est concentre pour obtenir une susbtanec sèche de 40-50 % poids/poids en pourcentage, en particulier 47-48% poids/poids en pourcentage.

14. Procédé selon la revendication 13, où la concentration s'obtient par évaporation, lyophilisation ou à pression réduit

15. Procédé selon l'une quelconque des revendications 11 à 14, où l'extrait torréfié contenant du XN est fermenté avec de la levure puis clarifié.

16. Procédé selon la revendication 15, dans lequel la clarification s'effectue par centrifugation ou filtration.

17. Procédé selon l'une quelconque des revendications 15 et 16, où la bière maltée torréfiée enrichie en XN est concentrée pour obtenir une teneur en extrait de 40-50 poids/poids en pourcentage, en particulier 47-48 % poids/poids en pourcentage.

18. Procédé selon la revendication 11, dans lequel l'extrait de houblon est predilué dans l'éthanol pour dissoudre le XN.

19. Procédé selon la revendication 18, où la dissolution du XN s'effectue par délayage, mélange, agitation, traitement à ultrasons et/ou traitement avec des appareils de dispersion.

20. Procédé selon l'une quelconque des revendications 18 et 19, **caractérisé en ce que** la dissolution du xanthohumol comprend en outre une cuisson et/ou l'application d'une tension alternative.

21. Procédé de fabrication d'un extrait aqueux contenant du XN obtenu à partir de produits torréfiés de céréales, malt de céréales ou de café, d'une teneur en XN supérieure à 10 mg/kg, comportant les phases suivantes:
a) Addition d'un produit à base de houblon contenant du XN à un extrait composé principalement ou entièrement de malts torréfiés, de malts colorés ou d'orge torréfiée; dans lequel le produit à base de houblon ajouté présente une teneur en XN qui, rapportée à sa masse intervenant dans sa fabrication correspond au moins au quintuple du houblon intervenant dans sa fabrication; et l'obtient après addition du produit à base de houblon contenant du XN à l'extrait torréfié aqueux, malgré une médiocre solubilité inhérente dans les solutions aqueuses, la dissolution, voire la dissolution intenseive du XN;
le procédé étant de plus **caractérisée en ce qu'**il comprend une ou plusieurs des phases suivantes:
b) mise en solution de XN par prédissolution du produit à base de houblon contenant du XN dans de l'éthanol:
c) mise en solution de XN par délayage, mélange, agitation, traitement aux ultrasons, traitement ultraturax ou autres forces mécaniques;
d) mise en solution de XN par cuisson de l'extrait torréfié;
e) mise en solution du XN par application d'une tension alternative,
f) addition du produit à base de houblon contenant du XN à hauteur d'au moins 50 mg de XN par litre.

22. Procédé de fabrication d'un extrait torréfie contenant du XN selon la revendication 21, en outre **caractérisé en ce que** l'extrait enrichi en XN est évaporé pour obtenir une substance sèche d'environ 50%.

23. Procédé de fabrication d'un extrait torréfié contenant du XN selon la revendication 21, en outre **caractérisé en ce que**, a) l'extrait enrichi en XN est fermenté à l'aide de levure, centrifugé ou filtré; b) la bière maltés torréfiée enrichie en XN est évaporée pour obtenir une teneur en extrait de 50% par exemple.

24. Procédé de fabrication d'un extrait torréfié contenant du XN selon la revendication 21 à 23, en outre **caractérisé en ce que** l'on utilise un extrait de malt torréfié en vente dans le commerce ou une bière maltée torréfiée en vente dans le commerce pour dissoudre l'extrait de houblon enrichi en XN.

25. Procédé de fabrication d'un extrait torréfié contenant du XN selon la revendication 21 ou 22, en outre **caractérisé en ce que** l'on utilise du café torréfié en vente dans le commerce ou du café soluble en vente dans le commerce pour dissoudre l'extrait de houblon enrichi en XN.

26. Extrait torréfié contenant du xanthohumol d'une teneur en XN supérieure à 10 mg/kg, dont la fabrication s'effectue selon les revendications 21 à 25. L'utilisation de cet extrait est prévue pour l'enrichissement naturel de XN dans des boissons comme la bière, les panachés, le vin, les boissons à base de café, l'ersatz de café ou une autre boisson sans alcool ou un précurseur de ces produits.

27. Extrait torréfié contenant du Xanthohumol d'une teneur en XN supérieure à 10 mg/kg, dont la fabrication s'effectue selon l'une des revendications 21 à 25. L'utilisation de cet extrait est prévue pour la fabrication de pâtisseries contenant du xanthohumol, de produits à tartiner, de desserts, de glaces, de viandes, de sauces et produits similaires ou un précurseur de ces produits.

28. Extrait torréfié contenant du xanthohumol d'une teneur en XN supérieure à 50 mg/kg, dont la fabrication s'effectue selon l'une des revendications 21 à 25.

29. Extrait torréfie contenant du xanthohumol d'une teneur en XN supérieure à 200 mg/kg, dont la fabrication s'effectue selon l'une des revendications 21 25.

30. Extrait torréfié contenant du xanthohumol d'une teneur en XN supérieure à 200 mg/kg.
